(19) Europäisches Patentamt / European Patent Office / Office européen des brevets

(11) **EP 4 403 202 A1**

(12) **EUROPEAN PATENT APPLICATION**
published in accordance with Art. 153(4) EPC

(43) Date of publication:
**24.07.2024 Bulletin 2024/30**

(21) Application number: **22870036.5**

(22) Date of filing: **15.09.2022**

(51) International Patent Classification (IPC):
**A61M 37/00** (2006.01)

(52) Cooperative Patent Classification (CPC):
**A61K 9/70; A61K 47/32; A61K 47/34; A61K 47/36; A61K 47/38; A61L 31/04; A61L 31/06; A61L 31/12; A61L 31/16; A61M 37/00**

(86) International application number:
**PCT/JP2022/034612**

(87) International publication number:
**WO 2023/042889 (23.03.2023 Gazette 2023/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.09.2021 JP 2021149939**

(71) Applicant: **Cosmed Pharmaceutical Co., Ltd.**
**Kyoto-shi, Kyoto 601-8014 (JP)**

(72) Inventors:
• **QUAN, Ying-shu**
**Kyoto-shi, Kyoto 601-8014 (JP)**
• **YAMASHITA, Hirofumi**
**Kyoto-shi, Kyoto 601-8014 (JP)**
• **KAJIYAMA, Kenji**
**Kyoto-shi, Kyoto 601-8014 (JP)**
• **LI, Ying-zhe**
**Kyoto-shi, Kyoto 601-8014 (JP)**
• **KAMIYAMA, Fumio**
**Kyoto-shi, Kyoto 601-8014 (JP)**

(74) Representative: **Ter Meer Steinmeister & Partner Patentanwälte mbB**
**Nymphenburger Straße 4**
**80335 München (DE)**

(54) **MICRONEEDLE ARRAY COATED WITH DRUG**

(57) To provide a practical microneedle preparation through finding an appropriate application condition regarding a geometric shape and a position on a needle of an applied material in a coated type microneedle. A microneedle array having a needle length of 350 um or more and 1,500 um or less and a diameter of a needle tip section apex of 15 um or more and 100 um or less, having a drug-applied portion, and comprising a non-biosoluble substance, preferably a non-biosoluble polymer, as a base, wherein no drug is applied to the needle tip section apex. It is preferable that the upper end of the drug-applied portion is a position 30 um or more lower than the needle tip section apex. The maximum diameter of the drug-applied portion is preferably 300 um or less.

[FIG. 2.]

| Shape condition | (1) Tip-applied (Comparative Example 1) | (2) Tip-non-applied (Example 1) |
|---|---|---|
| Image | | |
| Microscopic image | Length = 146.0µm  Length = 146.0µm  Length = 141 | Length = 144.4µm  Length = 144.4µm  Length = 144.4µm |
| Dimensions | Maximum diameter of applied portion: 140 to 150 µm  Length of applied portion: 200 µm | Maximum diameter of applied portion: 140 to 150 µm  Length of applied portion: 500 µm  Length of tip non-applied portion: 50 µm |
| Weight of applied portion (where (1) = 1.0) | 1.0 | 1.7 |

EP 4 403 202 A1

**Description**

TECHNICAL FIELD

The present invention relates to a microneedle array with a drug applied, and relates to the field of transdermal absorption preparations.

BACKGROUND ART

As a method of administering a medicine to a human body, oral administration and transdermal administration are often used. Injection is a typical transdermal administration method. However, injection is a procedure which takes time and labor of specialists such as physicians and nurses and is painful, so that many people do not welcome the procedure. On the other hand, a transdermal administration method without pain using a microneedle array has recently been attracting attention (Non-Patent Document 1).

In the transdermal administration of a medicine, skin stratum corneum works as a barrier to medicine permeation, so that enough permeability is not always provided by only applying the medicine on a skin surface. In contrast, perforation of stratum corneum by using a minute needle, i.e., a microneedle can remarkably improve medicine permeation efficiency compared to the application method. An article in which a large number of such microneedles are integrated on a substrate is a microneedle array. In addition, a product in which a pressure-sensitive adhesive sheet for adhering a microneedle array to a skin, a release sheet for protecting a pressure-sensitive adhesive surface, or the like is added to the microneedle array to facilitate its use is called a microneedle patch.

Some attempts to efficiently utilize expensive medicines are already known. Methods in which surfaces of microneedles are coated with a medicine by using a medicine solution (Patent Documents 1 to 4) and a method in which a medicine is granulated and the medicine is converged to the tips of microneedles by centrifugation while the microneedles maintain softness (Patent Document 5) have been reported. The method of coating the surfaces of microneedles with a medicine and the method of adhering a medicine to the tips of microneedles from a medicine solution have a problem that the medicine must be heated or that the adhered medicine falls away during insertion of the microneedles. In contrast, a method in which through dissolving a medicine in a solvent of a microneedle material, a microneedle body and a medicine adhered thereto are integrated together and the adhered medicine is prevented from falling away has been proposed (Patent Document 6).

The method of immersing the tips of microneedles into a medicine solution to adhere the medicine to the tips of microneedles is easily put into practical use due to its convenience (Patent Documents 1 to 4, 6). However, it is very difficult to quantitatively apply the medicine to the tips of microneedles with little unevenness.

In the case of microneedles made of a hydrophobic material, it is very difficult to apply a medicine from its aqueous solution. Whereas when microneedles made of a hydrophilic material are simply immersed into an aqueous medicine solution, the aqueous medicine solution extremely easily moves up among needles. Accordingly, it is extremely difficult to make a microneedle array hold a medicine quantitatively through immersing the microneedle array into an aqueous medicine solution to a certain fixed depth although many attempts have been repeated.

In order to prevent the capillary phenomenon, a method of masking any place other than the tip section of microneedles before applying a medicine has been proposed (Patent Document 2). A method of enhancing the quantitativeness of a medicine adhesion amount through putting a drug into a large number of holes and inserting microneedles into the holes (Patent Document 4) has also been proposed. However, it is very cumbersome to implement these methods.

In recent years, there also have been developed a microneedle provided with a step and maintaining a sharp tip section with a drug carried at the stepped part and with the tip of the microneedle exposed (Patent Document 7), and a technique of holding a medicine at a tip section and the step and thereby quantitatively administering the medicine (Patent Document 8). Furthermore, there also has been developed a technique in which, when a drug is applied to a microneedle having a specific length, the position of the lower end of the applied portion or the maximum diameter of the applied portion is adjusted to minimize a part of the drug to remain in the stratum corneum after skin administration (Patent Document 9).

PRIOR ART DOCUMENTS

PATENT DOCUMENTS

Patent Document 1: JP 2008-029710 A
Patent Document 2: JP 2007-521090 A

Patent Document 3: JP 2008-520370 A
Patent Document 4: WO 2008-139648
Patent Document 5: JP 2009-507573 A
Patent Document 6: JP 2011-224308 A
Patent Document 7: JP 2015-109963 A
Patent Document 8: JP 2014-079557 A
Patent Document 9: JP 2018-108375 A

NON-PATENT DOCUMENT

Non-Patent Document 1: Ying-shu Quan, Fumio Kamiyama, "The Course of Productization of Microneedle", The Archives of Practical Pharmacy, The Academy of Pharmaceutical Science and Technology, Japan, July 2009, Vol. 69, No. 4, pp. 272-276

SUMMARY OF THE INVENTION

PROBLEM TO BE SOLVED BY THE INVENTION

The present inventors actually applied a microneedle array with a drug applied to a microneedle tip section to an animal or a skin substitute (laminated parafilm), and evaluated the skin insertability of the microneedle in detail. The microneedle itself (before medicine application) is a circular, elliptical, or polygonal thin needle having a diameter of a needle tip section apex of 15 um or more and 100 um or less, a needle length of 350 um or more and 1,500 um or less, and an aspect ratio of 3 or more, and the microneedle itself has good skin insertability. However, when a medicine dissolved in a base is applied on the microneedle, the thickness of the needle increases and the skin insertability of the needle deteriorates. It has been found that the degree of the deterioration is greatly affected by the application amount of the medicine-containing base, the application position (a tip section or a position away from the tip), the applied portion diameter, and so on. An object of the present invention is to provide a practical microneedle preparation through finding an appropriate application condition regarding a geometric shape and a position on a needle of an applied material in a coated type microneedle.

MEANS FOR SOLVING THE PROBLEM

In order to solve the above problems, the present inventors have conducted detailed studies on an application amount, an application position, an application diameter, and the like of a drug for a wide variety of microneedles with a drug applied, and as a result of intensive studies on the skin insertability of a needle, have found that the intended object can be achieved by combining a microneedle having a specific shape with a specific ratio of a drug-applied portion, and have accomplished the present invention.
The present invention is as follows.

[1] A microneedle array having a needle length of 350 um or more and 1,500 um or less and a diameter of a needle tip section apex of 15 um or more and 100 um or less, having a drug-applied portion, and comprising a non-biosoluble substance as a material, wherein no drug is applied to the needle tip section apex.
[2] A microneedle array having a needle length of 350 um or more and 1,500 um or less and a diameter of a needle tip section apex of 15 um or more and 100 um or less, having a drug-applied portion, and comprising a non-biosoluble polymer as a base, wherein no drug is applied to the needle tip section apex.
[3] The microneedle array according to [1] or [2], wherein an upper end of the drug-applied portion is a position 30 um or more lower than the needle tip section apex.
[4] The microneedle array according to [3], wherein the drug-applied portion has a maximum diameter of 300 um or less.
[5] The microneedle array according to any one of [1] to [4], wherein the needle has an aspect ratio of 3 or more.
[6] The microneedle array according to any one of [1] to [5], wherein the amount of the drug applied per needle is 0.001 to 30 μg.
[7] The microneedle array according to any one of [2] to [6], wherein the non-biosoluble polymer is selected from the group consisting of nylon, polycarbonate, polylactic acid, poly(lactic acid-glycolic acid) copolymer, polyglycolic acid, polyethylene terephthalate, COP (cyclic olefin polymer), and mixtures thereof.
[8] The microneedle array according to any one of [2] to [6], wherein the non-biosoluble polymer is selected from the group consisting of hyaluronic acid, dextran, polyvinylpyrrolidone, sodium chondroitin sulfate, hydroxypropyl cellulose, polyvinyl alcohol, and mixtures thereof.

[9] The microneedle array according to any one of [1] to [8], wherein the drug-applied portion concomitantly contains a water-soluble substance selected from the group consisting of hyaluronic acid, collagen, dextrin, dextran, sodium chondroitin sulfate, hydroxypropyl cellulose, ethyl cellulose, carboxymethyl cellulose sodium salt, alginic acid, glucose, sucrose, maltose, trehalose, and mixtures thereof.

[10] A method for producing a microneedle array in which no drug is applied to a needle tip section apex, the method comprising:

a step of preparing a microneedle array having a needle length of 350 um or more and 1,500 um or less and a diameter of a needle tip section apex of 15 um or more and 100 um or less, and comprising a non-biosoluble polymer as a base; and

a step of applying a drug-containing application liquid having a solution viscosity of 50 to 2,000 mPa·s (25°C) to the microneedle array, and drying the drug-containing application liquid with such a setting that an upper end of a drug-applied portion after a drying step is a position 30 um or more lower than an upper end of a needle.

The microneedles installed in the microneedle array of the present invention each have a shape with a needle length of 350 um or more and 1,500 um or less and a diameter of a tip section apex of 15 pm or more and 100 pm or less. The needle itself (the needle before drug application) preferably has an aspect ratio of 3 or more, and specifically, the needle itself is a circular, elliptical, or polygonal thin needle, and the needle density is 50 to 1,500 needles/cm$^2$.

The microneedle array of the present invention may be one comprising a non-biosoluble substance as a material. For example, titanium, stainless steel, or the like can be used. In particular, it is appropriate that the microneedle array of the present invention comprises a non-biosoluble polymer as a base. Herein, the non-biosoluble polymer refers to a polymer having a property not to completely dissolve for at least 15 minutes after being molded into a microneedle and inserted into a skin. In the present invention, a polymer capable of being easily injection-molded or press-molded is preferable, and examples thereof include a polymer selected from the group consisting of nylon, polycarbonate, polylactic acid, poly(lactic acid-glycolic acid) copolymer, polyglycolic acid, polyethylene terephthalate, COP (cyclic olefin polymer), and mixtures thereof.

Alternatively, the base of the microneedle array of the present invention may be a polymer selected from the group consisting of hyaluronic acid, dextran, polyvinylpyrrolidone, sodium chondroitin sulfate, hydroxypropyl cellulose, polyvinyl alcohol, and mixtures thereof as long as the polymer has a property not to completely dissolve for at least 15 minutes after being molded into a microneedle and inserted into a skin.

The microneedle array of the present invention is characterized in that the upper end of the drug-applied portion is at a position 30 um or more lower than the upper end of the needle. That is, there is no drug at the upper end section of the microneedle with a drug applied, and it is clear that the upper end section of the microneedle is not coated.

The lower end and the upper end of the drug-applied portion are values obtained by measuring the lower end and the upper end of the microneedle with the drug applied, respectively, in a vertical direction from the substrate of the microneedle array. The length of the drug-applied portion is expressed by the difference between the lower end and the upper end of the microneedle with the drug applied.

EFFECT OF THE INVENTION

Among transdermal absorption preparations, a microneedle array with a drug applied to a needle tip section significantly improves a medicine permeation efficiency by piercing a skin stratum corneum acting as a barrier to medicine permeation during transdermal administration of the drug. However, it has been unexpectedly found that the application position of the drug has an important role on the skin insertability of the needle, and thus on the skin delivery property of the medicine. The medicine-coated microneedle array containing no drug at the needle upper end and retaining the sharp and thin shape of the microneedle has exhibited extremely good skin insertability. In contrast, it has been found that a microneedle array having an applied material on a needle upper portion receives large skin resistance at the time of insertion, resulting in poor skin insertability of the needle. The microneedle array of the present invention has succeeded in reducing the residue of the drug in the stratum corneum through specifying the shape of the microneedle and the position of the drug-applied portion. The microneedle array of the present invention is a transdermal absorption preparation with further enhanced transdermal drug delivery efficiency. The microneedle array of the present invention can deliver a rare medicine to a dermis layer with less burden on the body and transfer the medicine to blood to maintain a blood medicine concentration.

BRIEF DESCRIPTION OF THE DRAWINGS

FIG. 1 is a schematic diagram of a microneedle having a step.

FIG. 2 is images illustrating an example in which a drug is applied to a microneedle tip and an example in which a drug is not applied to a microneedle tip, and microscopic images corresponding to Comparative Example 1 and Example 1.

FIG. 3 is a micrograph of the microneedle array used in Example 1.

FIG. 4 is a micrograph of the microneedle array used in Examples 2 to 5.

Fig. 5 is a micrograph of a microneedle tip after application of the drug of Example 6.

FIG. 6 is a micrograph of a microneedle tip after application of the drug of Comparative Example 2.

FIG. 7 is a micrograph of a microneedle before application of the drug of Comparative Example 3.

FIG. 8 is a micrograph of a microneedle before application of the drug of Example 7.

FIG. 9 is a micrograph of a microneedle before application of the drug of Comparative Example 4.

## MODE FOR CARRYING OUT THE INVENTION

### Shape of microneedle

Each microneedle constituting the microneedle array has a needle length of 350 um or more and 1,500 um or less, preferably 400 um or more and 1,000 um or less, in order to ensure transdermal absorption of a medicine. A needle length of less than 350 um is unsuitable because it leads to a narrow applied portion. If the needle length exceeds 1,500 um, there is a risk of bleeding. The diameter of the tip section apex of the needle is 15 pm or more and 100 pm or less, preferably 20 pm or more and 60 pm or less in order to facilitate insertion into the skin and reduce the medicine residue in the skin. If the diameter of the tip section is less than 15 um, the tip section is weak, and when the microneedle is inserted into the skin, the tip section may bend due to skin resistance, resulting in defective insertion. If the diameter of the tip section exceeds 100 um, the tip section is excessively large and it has difficulty in insertion into the skin.

The needle itself (the needle before medicine application) has an aspect ratio of 3 or more, and the needle is a thin needle with a shape of the bottom face (the outer shape of the bottom face) of the needle being circular, elliptical, or polygonal. When the aspect ratio is less than 3, the microneedle is sharply thickened from the tip section, and thus there is a risk that the microneedle is pushed back from the skin in a short time even if the microneedle is once inserted into the skin by impactive administration. The aspect ratio of the needle is determined by the following formula.

$$\text{Aspect ratio of needle} = A/B$$

A: Needle length (um)

B: Diameter of needle root section (um)

The diameter (B) of the needle root section means the diameter of a circle when the shape of the needle root section (the shape of the bottom face of the needle) is circular, means the major axis of an ellipse when the shape of the needle root section (the shape of the bottom face of the needle) is elliptical, and means the length of a diagonal line of a polygon when the shape of the needle root section (the shape of the bottom face of the needle) is polygonal.

The needle density is 50 to 1,500 needles/$cm^2$. When the needle density is less than 50 needles/$cm^2$, the medicine application amount is small, and when the needle density exceeds 1,500 needles/$cm^2$, a problem often occurs in insertion of the needle into the skin due to the high density.

Each microneedle is in a cylindrical or conical shape with a circle bottom face, or in an elliptical cylindrical or elliptical conical shape with an elliptical bottom face. When the size of the ellipse is expressed, it is expressed using the major axis as a diameter, and the minor axis is shorter than the major axis as long as an ellipse can be formed. Microneedles having these shapes may have a step.

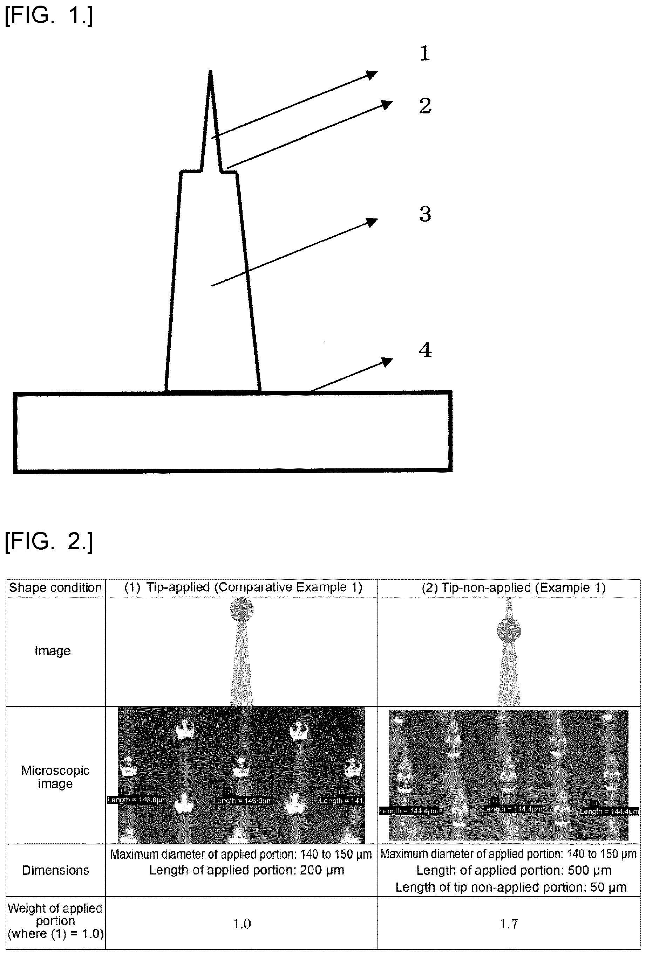

Herein, the step refers to a portion where the cross-sectional area of the microneedle is discontinuously reduced from a certain point of the microneedle toward the tip and where a cross section has a stepped shape as shown in FIG. 1. This example is a two-stage stepped microneedle. The shape of the stepped microneedle will be described with reference to FIG. 1. In the stepped microneedle, it is preferable that, of the needle length of 300 to 1,500 pm, a length of a tip section is 50 to 450 $\mu$m and the rest is a bottom section. The size of the margin 2 of the step between the tip section and the bottom section is preferably larger than 10 um and smaller than 100 um. The size of the margin is more preferably 14 to 50 um. Reference numeral 4 denotes a substrate of the microneedle array. FIG. 1 illustrates an example of a two-stage step, but there may be more steps. In a three-stage stepped microneedle, it is preferable that the microneedle has a tip section, an intermediate section, and a bottom section, and each of them has a length of 50 to 450 um.

The margin 2 of the step is a face perpendicular to the axis of the microneedle (a face parallel to the substrate) within a range of machining accuracy. In addition, the size of the margin 2 of the step refers to a difference in radius between the tip section and the bottom section on the stepped part. The tip section may be in a conical shape, a cylindrical shape, an elliptical cylindrical shape, or an elliptical conical shape depending on the shape of the microneedle. Similarly, the bottom section may be a circular truncated cone, a circular cylinder, an elliptic cylinder, or an elliptic truncated cone depending on the shape of the microneedle.

Microneedle having drug-applied portion

The microneedle array of the present invention has a drug-applied portion. The drug-applied portion is characterized by not being a tip section of the microneedle but being at a position of 30 um or less from the tip section of the microneedle. In other words, when the tip of the microneedle is directed upward, the upper end of the drug-applied portion is at a position 30 um or more lower than the tip of the needle. The length of the drug-applied portion is typically 100 um or more and 800 um or less, and preferably 150 um or more and 600 um or less.

The length of the drug-applied portion is a value obtained by measuring the lower end and the upper end of the microneedle with the drug applied each in a vertical direction from the substrate of the microneedle array. The length of the drug-applied portion is a distance from the lower end of the drug-applied portion to the upper end of the drug-applied portion along the length direction of the microneedle.

On the other hand, the drug-applied portion varies in thickness depending on the drug-containing application liquid and the number of times of application. In the present invention, the thickness of the applied portion can be expressed by the maximum diameter in the vicinity of the center of the drug-applied portion. The diameter in the vicinity of the center of the drug-applied portion is larger than the diameter of the microneedle before the drug application.

The maximum diameter of the drug-applied portion is 300 um or less. The maximum diameter of the drug-applied portion is desirably 200 um or less, and more desirably 150 um or less. When the maximum diameter of the drug-applied portion is excessively large, it is difficult to insert the microneedle into the skin, whereas when the maximum diameter is excessively small, the application amount of the medicine is small, leading to a reduced in-vivo delivery amount of the medicine. The maximum diameter of the drug-applied portion may be 50 um or more, or may be 100 um or more.

The total weight of the applied material including the drug per needle is desirably 0.001 to 30 $\mu$g. The total weight of the drug applied per needle is desirably 0.001 to 30 $\mu$g.

The present inventors have found that, in addition to the position of the drug-applied portion of a microneedle, when the thickest diameter in the vicinity of the center of the drug-applied portion is within the prescribed range, the residue of the drug in the stratum corneum is reduced, and a practical microneedle array can be realized.

Drug to be applied to microneedle

The drug is not particularly limited as long as it is a drug conventionally used as a transdermal absorption preparation. Examples of the drug include antipyretic analgesic, steroidal antiinflammatory agent, vasodilator, antiarrhythmic agent, antihypertensive agent, local anesthetic, hormone, antihistamine, general anesthetic, sedative hypnotic agent, anti-temper tantrum agent, psychoneurosis agent, skeletal muscle relaxant, autonomic agent, antiparkinsonian, diuretic, vasoconstrictor, respiratory stimulant, narcotic, and antigenic components against pathogens (e.g., vaccine antigenic protein). The content of the medicinal ingredient can be appropriately set depending on the characteristic of the ingredient, the purpose of administration, the administration subject, the number of administrations, and the like. In addition, the drug may be a raw material for cosmetics, particularly a valuable component.

In the present description, a medicine refers to a substance having a pharmacological action, and a drug is as described above, but the terms "drug" and "medicine" can be used interchangeably.

Many of the drugs are low molecular weight compounds having a molecular weight of 600 or less, but high molecular weight drugs can also be used. Examples of preferable high molecular weight drugs include physiologically active peptides and derivatives thereof, nucleic acids, oligonucleotides, various antigenic proteins, bacteria, and viral fragments.

Examples of the physiologically active peptides and derivatives thereof include calcitonin, adrenocorticotropic hormone, epidermal growth factor (EGF), parathyroid hormone (PTH), hPTH (1 → 34), insulin, atrial natriuretic peptide, growth hormone, growth hormone-releasing hormone, endothelin, and salts thereof. These medicinal ingredients can be administered not only for humans but also for animals. Examples of the antigenic proteins include influenza virus antigen, Japanese encephalitis virus antigen, diphtheria, tetanus antigens, HBs surface antigen, and HBe antigen.

Production of microneedle array

The base of the microneedle is preferably a polymer capable of being easily injection-molded or press-molded, and examples thereof include nylon, polycarbonate, polylactic acid, poly(lactic acid-glycolic acid) copolymer, polyglycolic acid, polyethylene terephthalate, COP (cyclic olefin polymer), and mixtures thereof. Alternatively, the non-biosoluble polymer may be hyaluronic acid, dextran, polyvinylpyrrolidone, sodium chondroitin sulfate, hydroxypropyl cellulose, polyvinyl alcohol, or a mixture thereof as long as the non-biosoluble polymer has a property not to completely dissolve for at least 15 minutes after being molded into a microneedle and inserted into a skin.

The microneedle array can be mass-produced using a mold (a die). A microneedle array comprising an injection-moldable polymer as a base may be produced by injection-molding the base using a mold (e.g., the method described in JP 2003-238347 A, [0017] and [0018]). For the injection molding die, stainless steel, heat resistant steel, superalloy, or the like can be used. A typical die has cavities corresponding to microneedles at a density of 100 to 1000 cavities per square centimeter in order to form microneedle shapes. For the purpose of forming the cavities, a fine processing means such as a grinder can be used.

When hyaluronic acid, dextran, polyvinylpyrrolidone, sodium chondroitin sulfate, hydroxypropyl cellulose, polyvinyl alcohol, or a mixture thereof is used as a base, an aqueous solution of the base may be poured into a mold, dried, and then removed (e.g., the method described in JP 2009-273872 A, [0029] to [0031]).

According to the above and other known production methods, a microneedle array has a needle length of 350 pm or more and 1,500 um or less and a diameter of a needle tip section apex of 15 um or more and 100 um or less and comprising a non-biosoluble polymer as a base is prepared.

Drug-containing application liquid and application step

The drug is applied to the microneedle array by immersing the tips of microneedles in a drug-containing application liquid to hold the medicine on the tips of the microneedles. The drug-containing application liquid is typically an aqueous solution, but may contain a solvent other than water in order to dissolve the medicine. The medicine may be completely dissolved or may be dispersed in a solvent.

It is desirable that a coexistent substance is dissolved in the drug-containing application liquid in advance, and the medicine is held on the microneedles together with the coexistent substance during drying after application. The coexistent substance is required to be a substance that does not impair the stability of the medicine, and for example, water-soluble polymer substances such as hyaluronic acid, collagen, dextrin, dextran, sodium chondroitin sulfate, hydroxypropyl cellulose, ethyl cellulose, sodium carboxymethyl cellulose salt, and alginic acid, low molecular weight saccharides such as glucose, sucrose, maltose, and trehalose, or mixtures thereof are suitable. The viscosity of the application liquid is preferably 50 to 2,000 mPa·s (25°C). When the viscosity is excessively low, the application amount to the microneedles tends to be small. When the viscosity is excessively high, application itself becomes difficult due to a decrease in fluidity due to the high viscosity.

The solution viscosity is measured using a B-type viscometer and an HB2 spindle at a rotation speed of 30 rpm.

When the coexistent substance is only a water-soluble polymer substance, a dissolution time of a coating film in the skin may be long at transdermal administration of the microneedles. In contrast, a coating film made of only a low molecular weight saccharide has insufficient mechanical strength. Therefore, as the coexistent substance for the drug-containing application liquid into which the microneedles are to be immersed, a mixture of a water-soluble polymer and a low molecular weight saccharide is desirable. In this case, the proportion of the low molecular weight saccharide is desirably 80% by mass or less with respect to the total weight of the coexistent substances.

The concentration of the coexistent substance in the drug-containing application liquid is desirably 2% by mass to 50% by mass in consideration of the viscosity of the application liquid. At a concentration lower than 2% by mass, the viscosity of the drug-containing application liquid is low, and the application adhesion amount at the time of immersion is small. When the concentration is more than 50% by mass, medicine application is not stable.

The immersion time and the number of immersions can be set depending on the affinity between the base of the microneedles and the drug-containing application liquid, and the drug-containing application liquid is applied such that the diameter in the vicinity of the center of the drug-applied portion is a desired value. After the application, the drug-containing application liquid is dried until the solvent is evaporated. The drug-containing application liquid may be naturally dried or forcibly dried by blowing dry air, nitrogen gas or the like. In the application, the drug-containing application liquid is filled in the medicine solution reservoir, and the microneedle array is immersed from above to adhere the medicine to the needles. The application device needs to be capable of controlling the immersion depth with an accuracy of about 20 microns in order to strictly define the immersion depth of the microneedle array. Whether the drug-containing application liquid is applied concentratedly to the needle upper end section or applied to the lower portion is adjusted by the immersion distance and the immersion time in the drug-containing application liquid.

Transdermal absorption preparation

The transdermal absorption preparation of the present invention is composed of a microneedle array having a drug-applied portion.

The transdermal absorption preparation of the present invention is administered to an animal having a skin tissue. Herein, examples of the animal include fish, amphibians, reptiles, birds (e.g., poultries such as chickens, quails, turkeys, ducks, gooses, call ducks, and ostrich), and mammals. Mammals may or may not include humans. Examples of the mammals other than humans include: rodents such as mice, rats, hamsters, and guinea pigs; pets such as rabbits, cats, and dogs; domestic animals such as pigs, cows, horses, sheep, and goats; and primates such as monkeys, orangutans, gorillas, and chimpanzees. The transdermal absorption preparation of the present invention is preferably administered to mammals.

FIG. 2 is images illustrating an example in which a drug is applied to a microneedle tip and an example in which a drug is not applied to a microneedle tip, and photographs corresponding to Comparative Example 1 and Example 1.

EXAMPLES

Hereinafter, the present invention will be explained with reference to Examples, but the present invention is not limited to the Examples.

Example 1 and Comparative Example 1

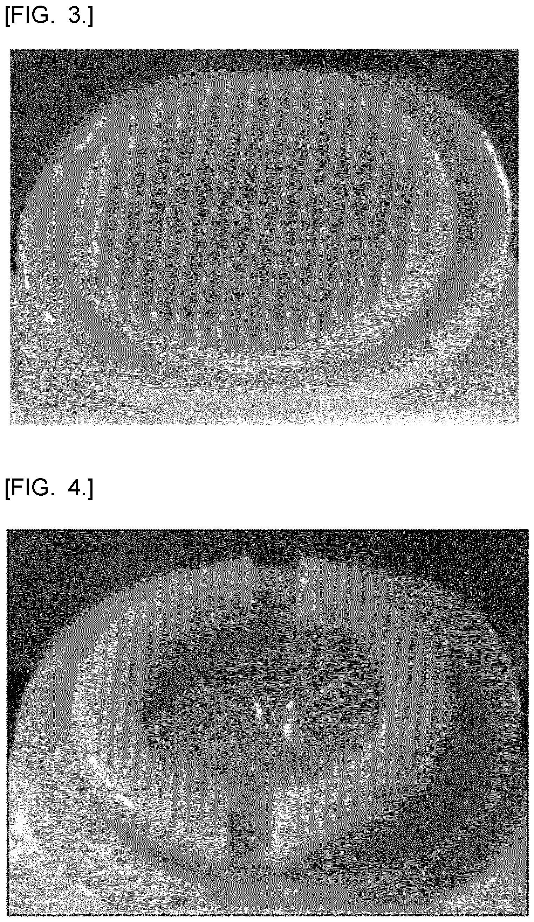

Polyglycolic acid (Kuredux, Kureha Corporation) as a raw material was injection-molded at an injection temperature of 260°C, yielding a microneedle array (PGA-MN). An overall image of the PGA-MN subjected to the experiment is shown in FIG. 3. In FIG. 3, the PGA-MN substrate has an elliptical shape with a major axis of 1.4 mm, a minor axis of 1.2 mm, and a thickness of 1.0 mm, and has a pedestal section with a thickness of 1.0 mm on an upper portion thereof. Each needle had a three-stage structure, had an overall length of 900 um, and also had a length of 300 um for each of the tip section, the intermediate section, and the root section. The total number of needles was 193. The diameter of the tip section was 40 um, and the diameter of the root section was 190 um.

The application liquid and the application conditions are as follows.

1) Hydroxypropyl cellulose, carboxymethyl cellulose sodium salt (CMC), dextran 70, and trehalose were mixed at a mass ratio of 1 : 1 : 1 : 3 to form a 30% by mass aqueous solution, and thus an application liquid was prepared.
2) Into the application liquid placed in a container were immersed the PGA-MN tips, and the application liquid was adhered to the needle tips.
3) The needle tips were then dried in a desiccator overnight. By varying the application depth and the application speed, the product of Comparative Example 1 and the product of Example 1 shown in FIG. 2 were produced. The non-applied portion in the product of Example 1 is immersed in the application liquid, and is not completely non-applied in a strict sense. As to "non-applied" referred to in the present invention, an application thickness as small as the thickness cannot be observed with an optical microscope, namely, less than 1 um, is also defined as non-applied. Test Example 1. Application of PGA-MN preparation to parafilm

1) Ten sheets of parafilm (thickness: 130 um), which are commonly used for evaluation of the insertability of microneedles, were stacked.
2) The product of Comparative Example 1 and the product of Example 1 shown in FIG. 2 were subjected to impact administration with an applicator.
3) The PGA-MN was removed and the through-holes of the parafilm were observed to evaluate the depth to which the PGA-MN had reached. The depth at which 80% or more of the total number of needles (193 needles) reached was defined as the reached depth.

Results: The product of Comparative Example 1 had a reached depth of 390 um, and the product of Example 1 had a reached depth of 520 um.

The product of Example 1 has a needle insertability better than that of the product of Comparative Example 1 although the weight of the applied portion is 1.7 times.

Test Example 2 Application of PGA-MN preparation to excised pig skin

1) The microneedles of the product of Comparative Example 1 and the product of Example 1 were administered to excised pig skins having a thickness of 2 mm.
2) The pig skins after the administration were observed by optical coherence tomography (OCT, THORABS, model: GANYMEDE), and the reached depth (insertion depth) of the needles was evaluated.

The results of four measurements and the average value are shown in Table 1.

[Table 1]

| | Insertion depth (um) | |
|---|---|---|
| | Comparative Example 1 | Example 1 |
| First time | 590 | 720 |
| Second time | 570 | 660 |
| Third time | 560 | 680 |
| Fourth time | 540 | 680 |
| Average value | 570 | 690 |

The product of Example 1 has a needle insertability to a pig skin better than that of the product of Comparative Example 1 although the weight of the applied portion is 1.7 times.

Examples 2 to 5

Polyglycolic acid (Kuredux, Kureha Corporation) as a raw material was injection-molded at an injection temperature of 260°C, yielding microneedles. An overall image of the PGA-MN subjected to the experiment is shown in FIG. 4. In FIG. 4, the PGA-MN substrate has an elliptical shape with a major axis of 1.4 mm, a minor axis of 1.2 mm, and a thickness of 1.0 mm, and has a pedestal section with a thickness of 1.0 mm on an upper portion thereof. The needles had a three-stage structure, the overall length was 900 um, and the total number of needles was 232. The diameter of the tip section was 40 um, and the diameter of the root section was 190 um. The same application liquid as in Example 1 was applied to the microneedles to prepare four types of microneedles as shown in Table 2. The viscosity was 260 mPa·s. The test of Test Example 1 (application to parafilm) was performed in the same manner as in Example 1, and the reached depth (insertion depth) was measured.

[Table 2]

| Measurement item | Maximum diameter of applied portion | Length of applied portion | Weight of base in applied material | Insertion depth |
|---|---|---|---|---|
| Unit | $\mu$m | $\mu$m | mg | $\mu$m |
| Example 2 | 95 | 460 | 110 | 650 |
| Example 3 | 110 | 490 | 245 | 650 |
| Example 4 | 125 | 495 | 440 | 520 |
| Example 5 | 145 | 450 | 580 | 390 |

These examples show that the thickness of the applied portion greatly affects the needle insertability.

Discussion

The experimental results revealed that the presence or absence of an applied material at the needle tip section greatly affects the skin insertability when the skin insertion is carried out under the same condition using the same microneedle. In these examples, microneedle administration by an applicator was performed under the spring condition specified by a spring constant of 0.26 N/mm and a length of 70 mm. In the case of an applicator having a smaller spring constant, the difference between the microneedle with the applied tip section and the non-applied microneedle was observed to be larger.
The present invention is considered to be important for obtaining a practical microneedle preparation.

Example 6 and Comparative Example 2

Microneedles were molded using polyglycolic acid (Kuredux, Kureha Corporation) as a raw material under the same production conditions as in Example 2. The shape and size of the molded microneedles were the same as those of the microneedle in Example 2 except that the needle tip section diameter was 28 um. The drug was applied using these microneedles. The application liquid was an aqueous solution containing 10% by mass of hyaluronic acid and 0.001% by mass of rhodamine B. The solution viscosity was 450 mPa·s (25°C). The tip of each of the microneedles was immersed in the application liquid placed in a container, pulled up, and dried to form an application layer. The maximum thickness of an applied portion was adjusted by varying the immersion distance and the number of immersions.

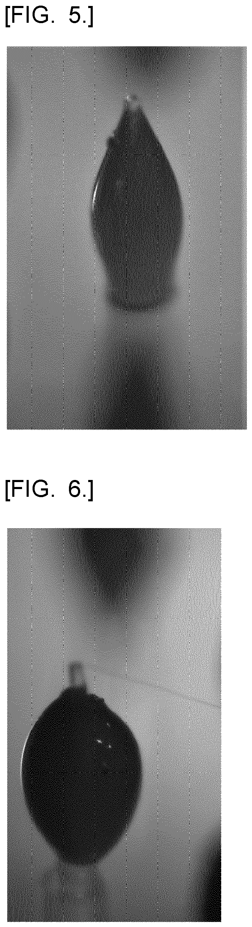

FIGS. 5 and 6 are micrographs of the applied microneedles having the maximum diameters of the applied portions of 240 um and 310 um, respectively. The main dimensions are summarized in Table 3.

Comparative Example 3

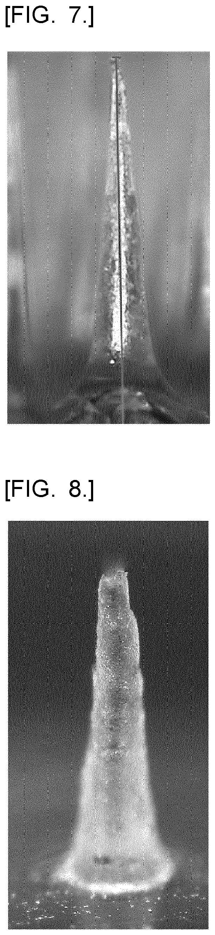

A 10% by mass solution of polylactic acid in acetone was poured into a dented mold having a thin tip section, then pressurized by centrifugation to form a needle, and then removed from the mold, affording a microneedle array. The tip thickness was 11 um. The application liquid was an aqueous solution containing 10% by mass of hyaluronic acid and 0.001% by mass of rhodamine B. The tip of each of the microneedles was immersed in the application liquid placed in a container, pulled up, and dried to form an application layer. A photograph of the microneedle before application is shown in FIG. 7.

Example 7 and Comparative Example 4

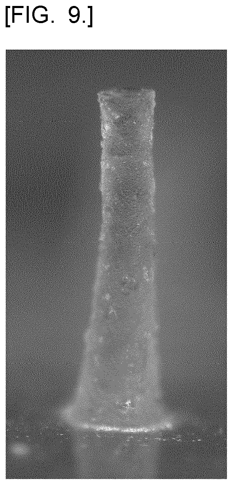

Molding of microneedle arrays having a thick tip section were performed in the same manner as in Comparative Example 3 except that the tip section of the dented mold was thick. The application conditions were the same as in Comparative Example 3. The photographs of the microneedles before application are shown in FIGS. 8 and 9.

[Table 3]

| Example/ Comparative Example | Overall length (pm) of needle | Diameter (pm) of needle tip section | Diameter (pm) of needle root section | Maximum diameter (pm) of applied portion | Length (pm) of applied portion | Number of needles/ microneedle array | Observation result after skin administration | Figure No. |
|---|---|---|---|---|---|---|---|---|
| Example 6 | 900 | 28 | 190 | 240 | 510 | 232 | (1) | 5 |
| Comparative Example 2 | 900 | 28 | 190 | 310 | 430 | 232 | (2) | 6 |
| Comparative Example 3 | 1,000 | 11 | 230 | 230 | 400 | 200 | (3) | 7 (before application) |
| Example 7 | 960 | 80 | 290 | 230 | 420 | 200 | (1) | 8 (before application) |
| Comparative Example 4 | 960 | 150 | 290 | 240 | 420 | 200 | (4) | 9 (before application) |

Test Example 3 Administration to excised pig skin of coated microneedles of five types of microneedle arrays

Five microneedle arrays of Examples 6 and 7 and Comparative Examples 2 to 4 were administered to excised pig skins in the same manner as in Test Example 2. At 10 minutes after the administration, each of the arrays was pulled up and the residual applied material on the array was observed and the results were summarized as follows.

(1) The applied portion of the needle entered the skin, and almost all of the applied material migrated into the skin.

(2) The applied portion had a large diameter and the entire applied portion of the needle did not enter the skin, so that much of the applied material remained on the needle.

(3) There were many needles whose tips were bent and did not enter the skin, so that most of the applied material remained on the needles.

(4) The needle tip section had a large diameter and only a part of the applied portion of the needle entered the skin, so that much of the applied material remained on the needle.

From the evaluation results of the pig skin permeability given in Table 3, it can be seen that regarding the maximum diameter of the applied portion (Example 6 vs. Comparative Example 2) and the diameter of the needle tip section apex (Example 6, Comparative Example 3, Example 7, and Comparative Example 4), the diameter of the needle tip section apex is preferably 15 um or more and 100 um or less, and the maximum diameter of the applied portion is preferably 300 um or less.

DESCRIPTION OF REFERENCE SYMBOLS

1    Tip section
2    Margin of step
3    Bottom section
4    Substrate of microneedle array

**Claims**

1. A microneedle array having a needle length of 350 um or more and 1,500 um or less and a diameter of a needle tip section apex of 15 um or more and 100 pm or less, having a drug-applied portion, and comprising a non-biosoluble substance as a material,
wherein no drug is applied to the needle tip section apex.

2. A microneedle array having a needle length of 350 um or more and 1,500 um or less and a diameter of a needle tip section apex of 15 um or more and 100 um or less, having a drug-applied portion, and comprising a non-biosoluble polymer as a base,
wherein no drug is applied to the needle tip section apex.

3. The microneedle array according to claim 1 or 2, wherein an upper end of the drug-applied portion is a position 30 um or more lower than the needle tip section apex.

4. The microneedle array according to claim 3, wherein the drug-applied portion has a maximum diameter of 300 um or less.

5. The microneedle array according to any one of claims 1 to 4, wherein the needle has an aspect ratio of 3 or more.

6. The microneedle array according to any one of claims 1 to 5, wherein the amount of the applied drug per needle is 0.001 to 30 $\mu$g.

7. The microneedle array according to any one of claims 2 to 6, wherein the non-biosoluble polymer is selected from the group consisting of nylon, polycarbonate, polylactic acid, poly(lactic acid-glycolic acid) copolymer, polyglycolic acid, polyethylene terephthalate, COP (cyclic olefin polymer), and mixtures thereof.

8. The microneedle array according to any one of claims 2 to 6, wherein the non-biosoluble polymer is selected from the group consisting of hyaluronic acid, dextran, polyvinylpyrrolidone, sodium chondroitin sulfate, hydroxypropyl cellulose, polyvinyl alcohol, and mixtures thereof.

9. The microneedle array according to any one of claims 1 to 8, wherein the drug-applied portion concomitantly contains a water-soluble substance selected from the group consisting of hyaluronic acid, collagen, dextrin, dextran, sodium chondroitin sulfate, hydroxypropyl cellulose, ethyl cellulose, carboxymethyl cellulose sodium salt, alginic acid, glucose, sucrose, maltose, trehalose, and mixtures thereof.

10. A method for producing a microneedle array in which no drug is applied to a needle tip section apex, the method comprising:

   a step of preparing a microneedle array having a needle length of 350 um or more and 1,500 um or less and a diameter of a needle tip section apex of 15 um or more and 100 um or less, and comprising a non-biosoluble polymer as a base; and
   a step of applying a drug-containing application liquid having a solution viscosity of 50 to 2,000 mPa·s (25°C) to the microneedle array, and drying the drug-containing application liquid with such a setting that an upper end of a drug-applied portion after a drying step is a position 30 um or more lower than an upper end of a needle.

[FIG. 1.]

[FIG. 2.]

| Shape condition | (1) Tip-applied (Comparative Example 1) | (2) Tip-non-applied (Example 1) |
|---|---|---|
| Image | | |
| Microscopic image | Length = 146.8μm   Length = 146.0μm   Length = 141. | Length = 144.4μm   Length = 144.4μm   Length = 144.4μm |
| Dimensions | Maximum diameter of applied portion: 140 to 150 μm<br>Length of applied portion: 200 μm | Maximum diameter of applied portion: 140 to 150 μm<br>Length of applied portion: 500 μm<br>Length of tip non-applied portion: 50 μm |
| Weight of applied portion (where (1) = 1.0) | 1.0 | 1.7 |

[FIG. 3.]

[FIG. 4.]

[FIG. 5.]

[FIG. 6.]

[FIG. 7.]

[FIG. 8.]

EP 4 403 202 A1

[FIG. 9.]

18

## INTERNATIONAL SEARCH REPORT

| International application No. |
|---|
| **PCT/JP2022/034612** |

### A. CLASSIFICATION OF SUBJECT MATTER

*A61M 37/00*(2006.01)i
FI:   A61M37/00 520; A61M37/00 505

According to International Patent Classification (IPC) or to both national classification and IPC

### B. FIELDS SEARCHED

Minimum documentation searched (classification system followed by classification symbols)

A61M37/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2014-514022 A (3M INNOVATIVE PROPERTIES CO.) 19 June 2014 (2014-06-19) paragraphs [0047], [0049], [0061], [0074], [0077], [0081], [0082], [0089], [0092], fig. 1, 3 | 1-10 |
| X | WO 2013/162053 A1 (TEIJIN LTD.) 31 October 2013 (2013-10-31) page 4, line 20 to page 14, line 8, fig. 1, 3 | 1-10 |
| X | JP 2015-109963 A (TEIJIN LTD.) 18 June 2015 (2015-06-18) paragraphs [0031]-[0040], fig. 2, 3 | 1-10 |
| A | WO 2018/124290 A1 (COSMED PHARMACEUTICAL CO., LTD.) 05 July 2018 (2018-07-05) entire text, all drawings | 1-10 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **13 October 2022** | **25 October 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/034612**

| Patent document cited in search report | | | Publication date (day/month/year) | Patent family member(s) | | | Publication date (day/month/year) |
|---|---|---|---|---|---|---|---|
| JP | 2014-514022 | A | 19 June 2014 | US 2014/0066843 A1 paragraphs [0107], [0109], [0121], [0134], [0137], [0141], [0142], [0149], [0152], fig. 1, 3 CN 103429222 A KR 10-2014-0010425 A WO 2012/122163 A1 | | | |
| WO | 2013/162053 | A1 | 31 October 2013 | US 2015/0094648 A1 paragraphs [0049]-[0092], fig. 1, 3 EP 2842595 A1 CN 104321105 A KR 10-2015-0003745 A | | | |
| JP | 2015-109963 | A | 18 June 2015 | (Family: none) | | | |
| WO | 2018/124290 | A1 | 05 July 2018 | US 2019/0344062 A1 entire text, all drawings EP 3563900 A1 CN 110035792 A KR 10-2019-0102178 A | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008029710 A
- JP 2007521090 A
- JP 2008520370 A
- WO 2008139648 A
- JP 2009507573 A
- JP 2011224308 A
- JP 2015109963 A
- JP 2014079557 A
- JP 2018108375 A
- JP 2003238347 A
- JP 2009273872 A

**Non-patent literature cited in the description**

- **YING-SHU QUAN ; FUMIO KAMIYAMA.** The Course of Productization of Microneedle. *The Archives of Practical Pharmacy, The Academy of Pharmaceutical Science and Technology, Japan,* July 2009, vol. 69 (4), 272-276